# EUROPEAN PATENT SPECIFICATION

(11) **EP 1 758 633 B1**
(45) Date of publication and mention of the grant of the patent: **18.03.2015**
(21) Application number: 05747644.2
(22) Date of filing: 04.05.2005
(51) Int. Cl.: A61M 25/00, A61K 9/16

(54) **METHODS FOR COMPOUNDING A THERAPEUTIC AGENT TO THE ADVENTITIA OF A VESSEL**
VERFAHREN FÜR DAS COMPOUNDIEREN EINES THERAPEUTIKUMS AN DIE ADVENTITIA EINES GEFÄSSES
PROCEDES DE MELANGE D'UN AGENT THERAPEUTIQUE DANS L'ADVENTICE D'UN VAISSEAU

(30) Priority: 13.05.2004 US 570691 P
(43) Date of publication of application: 07.03.2007
(73) Proprietor: Medtronic Vascular, Inc., Santa Rosa, CA 95403 (US)
(72) Inventor: TREMBLE, Patrice, Santa Rosa, CA 95405 (US); JUDD, Dianne, Minneapolis, MN 55410 (US); UDIPI, Kishore, Santa Rosa, CA 95403 (US); CHENG, Peiwen, Santa Rosa, CA 95409 (US); SUNDAR, Rangarajan (Ron), Torrance, CA 90503 (US); PATEL, Kaushik, Windsor, CA 95492 (US); TEDESCHI, Eugene, Santa Rosa, CA 95404 (US)
(74) Representative: Zimmermann, Gerd Heinrich
(86) International application number: PCT/US2005/015621
(87) International publication number: WO 2005/112569

(56) References cited:
- WO-A-02/49619
- WO-A-96/34599
- WO-A-98/30207
- US-A- 5 019 400
- US-A- 5 478 564
- US-A- 2002 022 055
- US-A- 2002 062 125
- US-A- 2003 055 446

## Description

### TECHNICAL FIELD

This invention relates generally to treatment of vascular conditions. More specifically, the invention relates to delivery of a therapeutic agent to the adventitia of a vessel for treatment of a vascular condition.

### BACKGROUND OF THE INVENTION

Heart disease, specifically coronary artery disease, is a major cause of death, disability, and healthcare expense in the United States and other industrialized countries. A wide variety of methods have been developed to provide treatment to diseased coronary arteries.

Studies have shown that delivering a therapeutic agent into the outer membrane of an artery, termed the tunica adventitia, adventitial layer, or simply adventitia, allows the agent to permeate the vessel. Thus the adventitia is capable of acting as a circulatory system within and for the artery.

A therapeutic agent may be delivered to the adventitia through the outer wall of the vessel by percutaneous injection or through the inner wall of the vessel by catheter delivery. For example, U.S. Patent Application Publication No. 2002/0022055 discloses a method for improving or increasing body passageway or cavity integrity that includes percutaneous delivery of a therapeutic agent by direct injection via an outer wall of the body passageway or cavity into the adventitia. The method includes applying a polymer or therapeutic agent/polymer complex to the external portion of the vessel as a periadventitial wrap. U.S. Patent Application Publication No. 2003/0077279 discloses a method for treating vascular disease by inhibiting toll-like receptor-4 (TLR-4). The method includes delivering a TLR-4 inhibiting composition by coating the composition onto a stent or by injecting the composition into the media and inner adventitia using an intravascular catheter.

Vascular delivery of therapeutic agents can be accomplished using a number of different delivery methods and devices. One such device is disclosed in U.S. Patent Application Publication No. 2003/0055446. This device includes an actuator joined to a distal end of a catheter. The actuator includes an expandable section designed to deploy a needle. When the expandable section is in an unactuated, furled condition, the needle is enclosed within the folds of the expandable section, preventing the needle from injuring the vessel walls while the catheter is being introduced into the target area of a vessel. Fluid connections are provided at the distal end of the catheter and a proximal end of the actuator to supply a therapeutic or diagnostic agent to the needle and to provide an activating fluid to the actuator. When actuated, the expandable section unfurls and expands, thrusting the needle outward and into a position approximately perpendicular to the vessel wall, thereby penetrating the vessel wall. When the activating fluid is removed, the expandable section returns to a furled state with the needle again enclosed within the folds of the expandable section to prevent trauma to the vessel during removal of the catheter.

While many therapeutic agents and their liquid carriers are suitable for delivery into the adventitia, many more are not. Because the adventitia is primarily fat and elastic fibers, lipophilic agents are particularly well absorbed and distributed. However, lipophilic agents typically require organic solvents that can cause cytotoxicity, hypersensitivity reactions, and other undesirable effects when delivered directly into tissue. Even therapeutic agents that are carried in a nontoxic fluid may damage the tissue of a vessel if their release is not controlled to prevent toxic levels accumulating within the tissue. Therefore, it would be desirable to have methods for compounding and delivering a therapeutic agent to the adventitia of a vessel that overcome the aforementioned and other disadvantages.

US 2002/0062125 describes a method of drug delivery to interstitial regions of the myocardium.

### SUMMARY OF THE INVENTION

Described herein is a method of delivering a therapeutic agent to the adventitia of a vessel using a catheter-based microsyringe. A therapeutic agent is formed into a plurality of microparticles. The microparticles are dispersed without dissolving throughout a pharmaceutically acceptable liquid carrier to form a therapeutic mixture or emulsion. A catheter is provided. The catheter includes a microsyringe operably attached to an actuator. The microsyringe includes a hollow needle in fluid communication with a therapeutic agent delivery conduit. The actuator is operable between an unactuated condition in which the needle is enclosed within the actuator and an actuated condition in which the needle is thrust outward by the actuator. The catheter is introduced into a target area of a vessel. The actuator is operated such that the needle is thrust outward and into a wall of the vessel. The therapeutic mixture is supplied to the therapeutic agent delivery conduit and delivered through the conduit to the needle and through the needle into the adventitia of the vessel. The actuator is operated such that the needle is withdrawn from the wall of the vessel and again enclosed within the actuator. The catheter is then removed from the vessel.

An aspect of the present invention is a method of compounding a therapeutic agent as defined in claim 1 for delivery to the adventitia of a vessel using a catheter-based microsyringe. A lipophilic therapeutic agent is formed into a plurality of microparticles. The microparticles are dispersed throughout an organic-solvent-free liquid carrier suitable for delivery to the adventitia of a vessel.

The aforementioned and other features and advantages of the invention will become further apparent from the following detailed description of the presently preferred embodiments, read in conjunction with the accompanying drawings. The detailed description and drawings are merely illustrative of the invention rather than limiting.

### BRIEF DESCRIPTION OF THE DRAWINGS

**FIG. 1** is a flow diagram of one embodiment of a method of delivering a therapeutic agent to the adventitia of a vessel using a catheter-based microsyringe;
**FIG. 2** is a schematic, perspective view of a catheter-based microsyringe;
**FIG. 3** is a transverse cross-section of an artery, showing the adventitia of the artery;
**FIG. 4** is a flow diagram of one embodiment of a method of compounding a therapeutic agent for delivery to the adventitia of a vessel using a catheter-based microsyringe, in accordance with the present invention.

### DETAILED DESCRIPTION OF THE PRESENTLY PREFERRED EMBODIMENTS

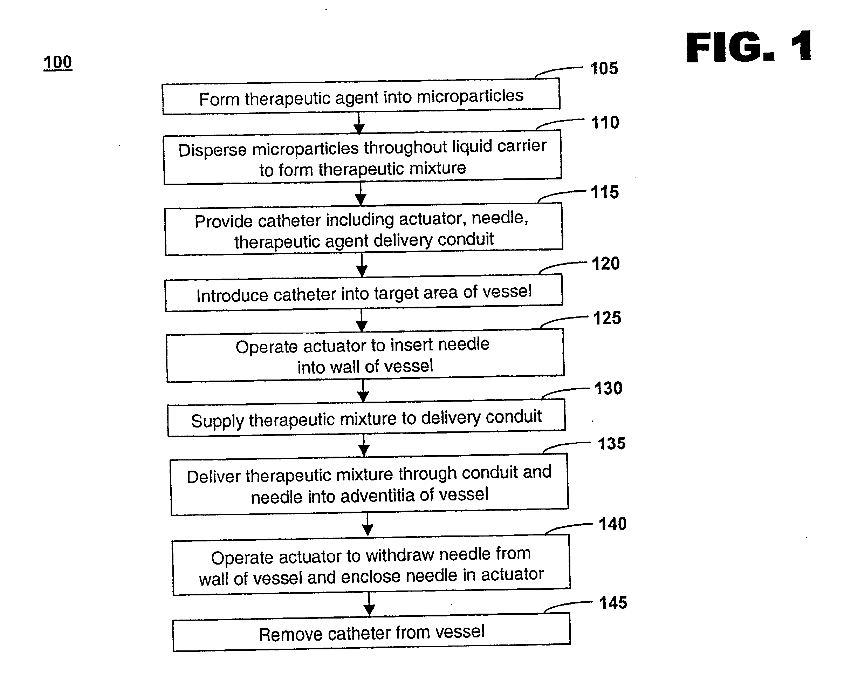

Described herein is a method of delivering a therapeutic agent to the adventitia of a vessel using a catheter-based microsyringe. **FIG. 1** shows a flow diagram of one example of the method at **100**.

A therapeutic agent is formed into a plurality of microparticles **(Block 105).** The therapeutic agent may include, for example, an antiproliferative agent, an antineoplastic agent, an antibiotic agent, an anti-inflammatory agent, an angiogenesis inhibitor, a metalloproteinase inhibitor, a serine proteinase inhibitor, molecules that block adhesion of lymphocytes or other immune response cells, combinations thereof, and the like. Microparticles of this invention may be nanoparticles or larger, e.g. up to 50 µm in diameter.

The microparticles may be formed by, for example, combining the therapeutic agent with a plurality of microspheres. The therapeutic agent may be encapsulated within the microspheres or attached to the outer surface of the microspheres, both techniques being known in the art. The microspheres may comprise either a biodegradable or a biocompatible matrix material. The matrix material may be a biodegradable polymer such as polylactide (PLA) or a biocompatible polymer such as a cellulose-based polymer. The matrix material may also be a protein such as albumin, a polysaccharide such as dextrans, or a lipid such as stearic acid. Where the matrix material is a lipid, the microsphere may be termed a liposome, i.e., a spherical particle formed by a lipid bilayer enclosing an aqueous compartment.

Alternatively, the therapeutic agent may be formed into dendrimers or carried as guest molecules within dendritic voids. A dendrimer is an artificially manufactured or synthesized molecule built up from branched monomers. Dendrimers have two major chemical environments: the surface chemistry due to the functional groups on the termination generation, which is the surface of the dendritic sphere; and the sphere's interior, the dendritic void, which is largely shielded from exterior environments due to the spherical shape of the dendrimer structure. Dendrimer research has confirmed the ability of dendrimers to accept guest molecules in the dendritic voids.

In yet another alternative, the microparticles may be formed into globules that comprise the discontinuous phase of an emulsion. By definition, the discontinuous phase of an emulsion is the dispersed liquid, and the continuous phase is the dispersion medium. In pharmaceutical preparations, forming a therapeutic agent into globules may include coating the globules with a gum or other mucilaginous substance.

The microparticles (regardless of how formed) are dispersed without dissolving into a pharmaceutically acceptable liquid carrier to form a therapeutic emulsion or mixture (Block 110). For a liquid carrier to be pharmaceutically acceptable for the present invention, it must be capable of being delivered directly into the adventitia without causing cytotoxicity, hypersensitivity reactions, or other undesirable effects. For example, a saline or other aqueous solution or, for an emulsion, a biocompatible dispersion medium would be an acceptable carrier. Organic solvents such as Cremaphor and ethanol would not be acceptable for the present invention.

Forming the therapeutic agent into microparticles permits some therapeutic agents that are lipophilic, to be dispersed throughout a nontoxic carrier fluid that is not otherwise a solvent for the therapeutic agent itself. Timed release in a highly controlled manner may also be facilitated by forming the therapeutic agent into microparticles. For example, where a therapeutic agent has been encapsulated within microspheres, the agent may be released over an extended period of time as a biodegradable polymer used as a matrix for the microspheres erodes or otherwise degrades, providing a continuous release of the agent while preventing it from reaching toxic levels. The agent may also be timed for release after a predetermined delay. Such timed release may be especially useful where the agent is a moderate to highly lipophilic or a hydrophilic therapeutic agent.

A catheter is provided **(Block 115).** The catheter includes a microsyringe, a microsyringe being a device for ejecting liquids through a small aperture. The microsyringe is operably attached to an actuator. The microsyringe includes a hollow needle in fluid communication with a therapeutic agent delivery conduit.

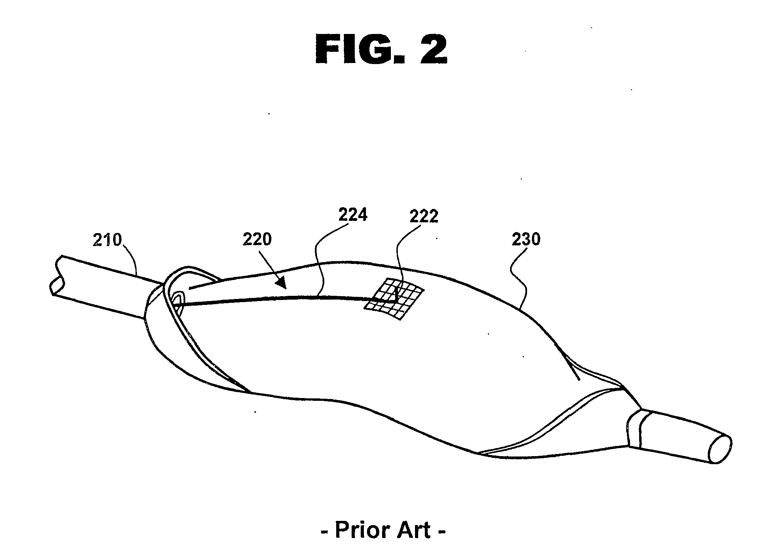

**FIG. 2** shows a schematic, perspective view of a catheter-based microsyringe which may be used. Catheter **210** includes a microsyringe **220** operably attached to an actuator **230.** Microsyringe **220** includes a hollow needle **222** in fluid communication with a therapeutic agent delivery conduit **224.** The actuator is positioned on a distal portion of the catheter and may comprise an inflatable balloon, as shown in this illustration. The present example is not limited to a particular microsyringe; however, the invention is especially useful with the device disclosed in U.S. Patent Application Publication No. 2003/0055446 A1.

The needle and delivery conduit are shown in **FIG. 2** as generally straight bodies. However, where the therapeutic mixture to be delivered is an emulsion, it may be desirable for a portion of either or both of the needle and the therapeutic agent delivery conduit to be tortuous, resulting in turbulence that mixes the emulsion prior to delivery. The emulsion may also be mixed prior to delivery by turbulence in the delivery conduit produced by two or more channels emptying into a single lumen within the delivery conduit.

The catheter is introduced into a target area of a vessel **(Block 120).** For example, a percutaneous access site may be created in the vessel to be treated or a vessel that leads to the vessel to be treated. A guidewire or a guiding catheter may be introduced through the percutaneous access site and advanced to a position adjacent to the target area of the vessel. The catheter including the microsyringe may be introduced into the vessel, either over a guidewire or directly into the guiding catheter. The catheter may then be guided to the target area of the vessel.

Once the catheter is in place, the actuator is operated to thrust the needle outward and into the wall of the vessel **(Block 125).** Where the actuator comprises an inflatable balloon, the act of inflating the balloon may move the needle into a position approximately perpendicular to the vessel wall, thereby thrusting the needle outward and inserting it into the vessel wall. The balloon is shown in **FIG. 2** in an actuated condition. Prior to operating the actuator, the needle may be held inside the folds of the uninflated balloon, preventing the needle from injuring the vascular walls while the catheter is being introduced into the target area of the vessel.

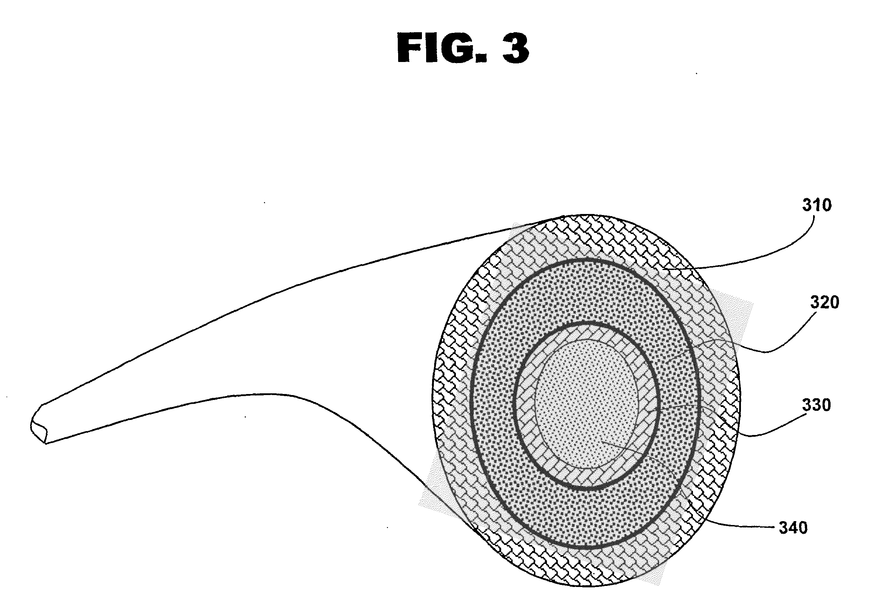

The therapeutic mixture is supplied to the therapeutic agent delivery conduit from, for example, a reservoir positioned outside the body of the individual undergoing therapy **(Block 130).** The therapeutic mixture is then delivered through the conduit into the needle and through the needle into the adventitia of the vessel **(Block 135).** **FIG. 3** is an illustration of an artery with the adventitia (also referred to as tunica adventitia or outer membrane) of the vessel indicated at **310.** The tunica media, tunica intima, and lumen are indicated at **320, 330,** and **340,** respectively. Studies have shown that delivering a therapeutic agent into the adventitia, which comprises fat and elastic fibers, allows the agent to permeate the vessel.

After delivery of the therapeutic agent into the adventitia, the actuator is operated to withdraw the needle from the wall of the vessel and enclose it within the actuator **(Block 140).** Where the actuator comprises an inflatable balloon, deflating the balloon may move the needle back inside the folds of the deflated balloon, thereby enclosing the needle and preventing trauma to the vessel during removal of the catheter from the vessel. The catheter may then be removed from the vessel **(Block 145).** As will be clear to one skilled in the art, the catheter may be repositioned and the steps for delivering the therapeutic agent to the adventitia repeated any number of times before removing the catheter from the vessel.

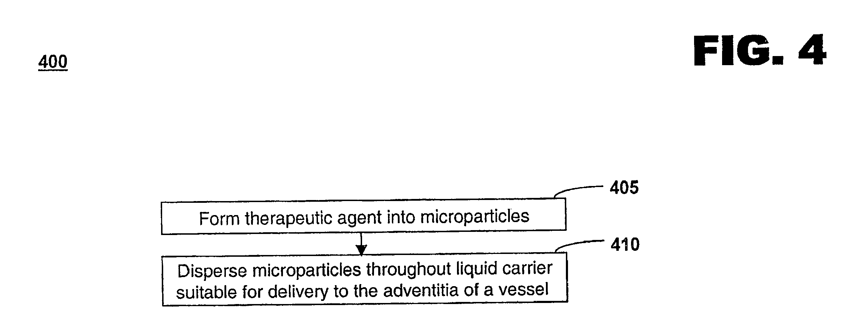

An aspect of the present invention is a method of compounding a therapeutic agent for delivery to the adventitia of a vessel using a catheter-based microsyringe. One embodiment of the method, in accordance with the present invention, is diagrammed in **FIG. 4** at **400.**

A therapeutic agent is formed into a plurality of microparticles **(Block 405).** The therapeutic agent may include, for example, an antiproliferative agent, an antineoplastic agent, an antibiotic agent, an anti-inflammatory agent, an angiogenesis inhibitor, a metalloproteinase inhibitor, a serine proteinase inhibitor, molecules that block adhesion of lymphocytes or other immune response cells, combinations thereof, and the like. Microparticles of this invention may be nanoparticles or larger, e.g. up to 50 µm in diameter.

The microparticles may be formed by, for example, combining the therapeutic agent with a plurality of microspheres. The therapeutic agent may be encapsulated within the microspheres or attached to the outer surface of the microspheres, both techniques being known in the art. The microspheres may comprise either a biodegradable or a biocompatible matrix material. The matrix material may be a biodegradable polymer such as polylactide (PLA) or a biocompatible polymer such as a cellulose-based polymer, for example ethyl cellulose, carboxymethylcellulose, cellulose acetate, methylcellulose or any other acceptable polymer. The matrix material may also be a protein such as albumin, a polysaccharide such as dextrans, or a lipid. Where the matrix material is a lipid, the microsphere may be termed a liposome, a spherical particle formed by a lipid bilayer enclosing an aqueous compartment.

Alternatively, the therapeutic agent may be formed into dendrimers or carried as guest molecules within dendritic voids. A dendrimer is an artificially manufactured or synthesized molecule built up from branched monomers. Dendrimers have two major chemical environments: the surface chemistry due to the functional groups on the termination generation, which is the surface of the dendritic sphere; and the sphere's interior, the dendritic void, which is largely shielded from exterior environments due to the spherical shape of the dendrimer structure. Dendrimer research has confirmed the ability of dendrimers to accept guest molecules in the dendritic voids.

In yet another alternative, the microparticles may be formed into globules that comprise the discontinuous phase of an emulsion. By definition, the discontinuous phase of an emulsion is the dispersed liquid, and the continuous phase is the dispersion medium. In pharmaceutical preparations, forming a therapeutic agent into globules may include coating the globules with a gum or other mucilaginous substance, including, without limitation, xanthan gum, carrageenan, gum arabic, guar gum.

The microparticles are dispersed throughout a liquid carrier suitable for delivery to the adventitia of a vessel **(Block 410).** For a liquid carrier to be suitable, it must be capable of being delivered directly into the adventitia without causing cytotoxicity, hypersensitivity reactions, or other undesirable effects. For example, a saline or other aqueous solution or, for an emulsion, a biocompatible dispersion medium would be an acceptable carrier. Organic solvents such as Cremaphor and ethanol would not be suitable for the present invention.

Forming the therapeutic agent into microparticles permits some therapeutic agents, for example those that are lipophilic, to be dispersed throughout a nontoxic carrier fluid that is not otherwise a solvent for the therapeutic agent. Timed release of a lipophilic or a hydrophilic therapeutic agent may also be achieved by forming the therapeutic agent into microparticles. For example, where a therapeutic agent has been encapsulated within microspheres, the agent may be released over an extended period of time as a biodegradable polymer used as a matrix for the microspheres erodes or otherwise degrades, providing a continuous release of the agent while preventing it from reaching toxic levels. The agent may also be timed for release after a predetermined delay.

It will be understood by those skilled in the art that the pharmaceutical composition formed by dispersing microparticles formed from a therapeutic agent within a liquid carrier suitable for delivery to the adventitia of a vessel can be used for the manufacture of a medicament for the treatment of coronary artery disease.

While the embodiments of the invention disclosed herein are presently considered to be preferred, various changes and modifications can be made without departing from the scope of the invention.

## Claims

1. A method of compounding a lipophilic therapeutic agent so that it is adapted for delivery to the adventitia of a vessel using a catheter-based microsyringe, comprising the steps of:
(a) forming a lipophilic therapeutic agent into a plurality of microparticles, wherein the lipophilic therapeutic agent includes an antiproliferative agent, an antineoplastic agent, an antibiotic agent, an anti-inflammatory agent, an angiogenesis inhibitor, a metalloproteinase inhibitor, a serine proteinase inhibitor, or any combinations thereof; and
(b) dispersing the microparticles throughout an organic-solvent-free liquid carrier adapted for delivery to the adventitia of the vessel.

2. The method according to claim 1, wherein the liquid carrier is a saline or other aqueous solution or, for an emulsion, a biocompatible dispersion medium.

3. The method according to any of the preceding claims, where the microparticles are nanoparticles.

4. The method according to any of claims 1 or 2, where the microparticles have a size of up to 50 µm in diameter.

5. The method according to any of the preceding claims, wherein the microparticles are formed by combining the therapeutic agent with a plurality of microspheres.

6. The method according to claim 5, wherein the lipophilic therapeutic agent is encapsulated within the microspheres.

7. The method according to claim 5, wherein the lipophilic therapeutic agent is attached to the outer surface of the microspheres.

8. The method according to any of claims 5 to 7, wherein the microspheres comprise a biodegradable or a biocompatible matrix material.

9. The method according to claim 8, wherein the matrix material is a biodegradable polymer.

10. The method according to claim 9, wherein the matrix material is polylactide (PLA).

11. The method according to claim 8, wherein the matrix material is a biocompatible cellulose-based polymer.

12. The method according to claim 11, wherein the matrix material is ethyl cellulose, carboxymethylcellulose, cellulose acetate, or methylcellulose.

13. The method according to claim 8, wherein the matrix material is a protein.

14. The method according to claim 13, wherein the matrix material is albumin.

15. The method according to claim 8, wherein the matrix material is a polysaccharide.

16. The method according to claim 15, wherein the matrix material is dextrans.

17. The method according to claim 8, wherein the matrix material is a liposome.

18. The method according to any of claims 1 to 4, wherein the lipophilic therapeutic agent is formed into a dendrimer.

19. The method according to any of claims 1 to 4, wherein the lipophilic therapeutic agent is carried as guest molecules within a dendritic void of a dendrimer.

20. The method according to any of claims 1 to 4, wherein the microparticles are formed into globules that comprise the discontinuous phase of an emulsion.

21. The method according to claim 20, further comprising the step of coating the globules with a gum or other mucilaginous substance.

22. The method according to claim 21, wherein the globules are coated with xanthan gum, carrageenan, gum arabic, or guar gum.

23. The method according to any of the preceding claims, wherein the liquid carrier is not a solvent for the lipophilic therapeutic agent.

24. A pharmaceutical composition, comprising
a plurality of microparticles, wherein the microparticles are adapted for delivery to the adventitia of a vessel using a catheter-based microsyringe, the microparticles comprising a lipophilic therapeutic agent, the lipophilic therapeutic agent being selected from the group consisting of an antiproliferative agent, an antineoplastic agent, an antibiotic agent, an anti-inflammatory agent, an angiogenesis inhibitor, a metalloproteinase inhibitor, a serine proteinase inhibitor, or any combinations thereof, and
an organic-solvent-free liquid carrier adapted for delivery to the adventitia of the vessel,
wherein the microparticles are dispersed in said liquid carrier for use as medicament.

25. The pharmaceutical composition of claim 24, wherein the microparticles have a size of up to 50 µm in diameter.

26. The pharmaceutical composition according to claim 24 or 25, wherein the lipophilic therapeutic agent is encapsulated within microspheres.

27. The pharmaceutical composition according to claim 24 or 25, wherein the lipophilic therapeutic agent is attached to the outer surface of microspheres.

28. The pharmaceutical composition according to any of claims 24 or 25, wherein the lipophilic therapeutic agent is formed into a dendrimer.

29. The pharmaceutical composition according to any of claims 24 or 25, wherein the lipophilic therapeutic agent is carried as guest molecules within a dendritic void of a dendrimer.

30. The pharmaceutical composition according to any of claims 24 or 25, wherein the microparticles are formed into globules that comprise the discontinuous phase of an emulsion.

31. The pharmaceutical composition according to any of claims 24 to 30, wherein the liquid carrier is a saline or other aqueous solution or, for an emulsion, a biocompatible dispersion medium.

32. The pharmaceutical composition according to any of claims 24 to 31, wherein a therapeutic agent is lipophilic and the liquid carrier is a saline or aqueous solution.

## Patentansprüche

1. Verfahren zum Compoundieren eines lipophilen therapeutischen Mittels, derart, dass es zur Abgabe an die Adventitia eines Gefäßes unter Verwendung einer Katheter-basierten Mikrospritze adaptiert ist, umfassend die Schritte:
(a) Formieren eines lipophilen therapeutischen Mittels zu einer Vielzahl von Mikropartikeln, wobei das lipophile therapeutische Mittel ein antiproliferatives Mittel, ein antineoplastisches Mittel, ein Antibiotikum, ein entzündungshemmendes Mittel, einen Angiogese-Inhibitor, einen Metalloproteinase-Inhibitor, einen Serinproteinase-Inhibitor oder eine Kombination davon umfasst; und
(b) Dispergieren der Mikropartikel in einem flüssigen Träger, der frei von organischen Lösungsmitteln ist, der für die Abgabe an die Adventitia des Gefäßes adaptiert ist.

2. Verfahren nach Anspruch 1, wobei der flüssige Träger eine Kochsalzlösung oder andere wässrige Lösung oder für eine Emulsion ein biokompatibles Dispersionsmedium ist.

3. Verfahren nach einem der vorhergehenden Ansprüche, wobei die Mikropartikel Nanopartikel sind.

4. Verfahren nach einem der Ansprüche 1 oder 2, wobei die Mikropartikel eine Größe von bis zu 50 µm im Durchmesser aufweisen.

5. Verfahren nach einem der vorhergehenden Ansprüche, wobei die Mikropartikel durch Kombinieren des therapeutischen Mittels mit einer Vielzahl von Mikrokügelchen formiert werden.

6. Verfahren nach Anspruch 5, wobei das lipophile therapeutische Mittel in den Mikrokügelchen eingekapselt ist.

7. Verfahren nach Anspruch 5, wobei das lipophile therapeutische Mittel an der äußeren Oberfläche der Mikrokügelchen gebunden ist.

8. Verfahren nach einem der Ansprüche 5 bis 7, wobei die Mikrokügelchen ein biologisch abbaubares oder ein biokompatibles Matrixmaterial umfassen.

9. Verfahren nach Anspruch 8, wobei das Matrixmaterial ein biologisch abbaubares Polymer ist.

10. Verfahren nach Anspruch 9, wobei das Matrixmaterial Polylactid (PLA) ist.

11. Verfahren nach Anspruch 8, wobei das Matrixmaterial ein biokompatibles Polymer auf Cellulosebasis ist.

12. Verfahren nach Anspruch 11, wobei das Matrixmaterial Ethylcellulose, Carboxymethylcellulose, Celluloseacetat oder Methylcellulose ist.

13. Verfahren nach Anspruch 8, wobei das Matrixmaterial ein Protein ist.

14. Verfahren nach Anspruch 13, wobei das Matrixmaterial Albumin ist.

15. Verfahren nach Anspruch 8, wobei das Matrixmaterial ein Polysaccharid ist.

16. Verfahren nach Anspruch 15, wobei das Matrixmaterial Dextrane ist.

17. Verfahren nach Anspruch 8, wobei das Matrixmaterial ein Liposom ist.

18. Verfahren nach einem der Ansprüche 1 bis 4, wobei das lipophile therapeutische Mittel zu einem Dendrimer formiert wird.

19. Verfahren nach einem der Ansprüche 1 bis 4, wobei das lipophile therapeutische Mittel als Gastmoleküle innerhalb eines dendritischen Hohlraums eines Dendrimers getragen wird.

20. Verfahren nach einem der Ansprüche 1 bis 4, wobei die Mikropartikel zu Kügelchen formiert werden, die die diskontinuierliche Phase einer Emulsion umfassen.

21. Verfahren nach Anspruch 20, weiter umfassend den Schritt des Beschichtens der Kügelchen mit einem Gummi oder einer anderen schleimartigen Substanz.

22. Verfahren nach Anspruch 21, wobei die Kügelchen mit Xanthangummi, Carrageen, Gummi arabicum oder Guargummi beschichtet werden.

23. Verfahren nach einem der vorhergehenden Ansprüche, wobei der flüssige Träger kein Lösungsmittel für das lipophile therapeutische Mittel ist.

24. Pharmazeutische Zusammensetzung, umfassend
eine Vielzahl von Mikropartikeln, wobei die Mikropartikel zur Abgabe an die Adventitia eines Gefäßes unter Verwendung einer Katheter-basierten Mikrospritze adaptiert sind, wobei die Mikropartikel ein lipophiles therapeutisches Mittel umfassen und das lipophile therapeutische Mittel ausgewählt ist aus der Gruppe bestehend aus einem antiproliferativen Mittel, einem antineoplastischen Mittel, einem Antibiotikum, einem entzündungshemmendem Mittel, einem Angiogese-Inhibitor, einem Metalloproteinase-Inhibitor, einem Serinproteinase-Inhibitor oder einer Kombination davon; und
einen flüssigen Träger, der frei von organischen Lösungsmitteln ist und für die Abgabe an die Adventitia des Gefäßes adaptiert ist,
wobei die Mikropartikel in dem flüssigen Träger zur Verwendung als Medikament dispergiert sind.

25. Pharmazeutische Zusammensetzung nach Anspruch 24, wobei die Mikropartikel eine Größe von bis zu 50 µm im Durchmesser aufweisen.

26. Pharmazeutische Zusammensetzung nach Anspruch 24 oder 25, wobei das lipophile therapeutische Mittel in Mikrokügelchen eingekapselt ist.

27. Pharmazeutische Zusammensetzung nach Anspruch 24 oder 25, wobei das lipophile therapeutische Mittel an der äußeren Oberfläche von Mikrokügelchen gebunden ist.

28. Pharmazeutische Zusammensetzung nach einem der Ansprüche 24 oder 25, wobei das lipophile therapeutische Mittel zu einem Dendrimer formiert ist.

29. Pharmazeutische Zusammensetzung nach einem der Ansprüche 24 oder 25, wobei das lipophile therapeutische Mittel als Gastmoleküle innerhalb eines dendritischen Hohlraums eines Dendrimers getragen wird.

30. Pharmazeutische Zusammensetzung nach einem der Ansprüche 24 oder 25, wobei die Mikropartikel zu Kügelchen formiert werden, die die diskontinuierliche Phase einer Emulsion umfassen.

31. Pharmazeutische Zusammensetzung nach einem der Ansprüche 24 bis 30, wobei der flüssige Träger eine Kochsalzlösung oder andere wässrige Lösung oder für eine Emulsion ein biokompatibles Dispersionsmedium ist.

32. Pharmazeutische Zusammensetzung nach einem der Ansprüche 24 bis 31, wobei ein therapeutisches Mittel lipophil ist und der flüssige Träger eine Kochsalzlösung oder eine wässrige Lösung ist.

## Revendications

1. Procédé de mélange d'un agent thérapeutique lipophile de sorte qu'il soit adapté à l'administration à l'adventice d'un vaisseau en utilisant une microseringue à base de cathéter, comprenant les étapes consistant à :
(a) former un agent thérapeutique lipophile dans une pluralité de microparticules, dans laquelle l'agent thérapeutique lipophile comprend un agent antiprolifératif, un agent antinéoplasique, un agent antibiotique, un agent anti-inflammatoire, un inhibiteur de l'angiogenèse, un inhibiteur de métalloprotéinase, un inhibiteur de sérine protéinase, ou toutes combinaisons de ceux-ci ;
et à
(b) disperser les microparticules à travers un support liquide sans solvant organique adapté pour l'administration à l'adventice du vaisseau.

2. Procédé selon la revendication 1, dans lequel le support liquid est une solution saline ou une autre solution aqueuse ou, pour une émulsion, un milieu de dispersion biocompatible.

3. Procédé selon l'une quelconque des revendications précédentes, où les microparticules sont des nanoparticules.

4. Procédé selon l'une quelconque des revendications 1 ou 2, où les microparticules ont une taille allant jusqu'à 50 µm de diamètre.

5. Procédé selon l'une quelconque des revendications précédentes, dans lequel les microparticules sont formées en combinant l'agent thérapeutique avec une pluralité de microsphères.

6. Procédé selon la revendication 5, dans lequel l'agent thérapeutique lipophile est encapsulé à l'intérieur des microsphères.

7. Procédé selon la revendication 5, dans lequel l'agent thérapeutique lipophile est fixé à la surface extérieure des microsphères.

8. Procédé selon l'une quelconque des revendications 5 à 7, dans lequel les microsphères comprennent un matériau de matrice biodégradable ou biocompatible.

9. Procédé selon la revendication 8, dans lequel le matériau de matrice est un polymère biodégradable.

10. Procédé selon la revendication 9, dans lequel le matériau de matrice est un polylactide (PLA).

11. Procédé selon la revendication 8, dans lequel le matériau de matrice est un polymère à base de cellulose biocompatible.

12. Procédé selon la revendication 11, dans lequel le matériau de matrice est l'éthyle cellulose, la carboxyméthylcellulose, l'acétate de cellulose, ou la méthylcellulose.

13. Procédé selon la revendication 8, dans lequel le matériau de matrice est une protéine.

14. Procédé selon la revendication 13, dans lequel le matériau de matrice est l'albumine.

15. Procédé selon la revendication 8, dans lequel le matériau de matrice est un polysaccharide.

16. Procédé selon la revendication 15, dans lequel le matériau de matrice sont les dextranes.

17. Procédé selon la revendication 8, dans lequel le matériau de matrice est un liposome.

18. Procédé selon l'une quelconque des revendications 1 à 4, dans lequel l'agent thérapeutique lipophile est formé en un dendrimère.

19. Procédé selon l'une quelconque des revendications 1 à 4, dans lequel l'agent thérapeutique lipophile est transporté sous la forme de molécules hôtes à l'intérieur d'un vide dendritique d'un dendrimère.

20. Procédé selon l'une quelconque des revendications 1 à 4, dans lequel les microparticules sont formées dans des globules qui comprennent la phase discontinue d'une émulsion.

21. Procédé selon la revendication 20, comprenant en outre l'étape consistant à recouvrir les globules de gomme ou d'une autre substance mucilagineuse.

22. Procédé selon la revendication 21, dans lequel les globules sont recouverts de gomme de xanthane, de carraghénane, de gomme arabique, ou de gomme de guar.

23. Procédé selon l'une quelconque des revendications précédentes, dans lequel le support liquide n'est pas un solvant pour l'agent thérapeutique lipophile.

24. Composition pharmaceutique, comprenant
une pluralité de microparticules, dans laquelle les microparticules sont adaptées pour l'administration à l'adventice d'un vaisseau en utilisant une microseringue à base de cathéter, les microparticules comprenant un agent thérapeutique lipophile, l'agent thérapeutique lipophile étant sélectionné dans le groupe constitué d'un agent antiprolifératif, d'un agent antinéoplasique, d'un agent antibiotique, d'un agent anti-inflammatoire, d'un inhibiteur de l'angiogenèse, d'un inhibiteur de métalloprotéinase, d'un inhibiteur de sérine protéinase, ou de toutes combinaisons de ceux-ci ; et
un support liquide sans solvant organique adapté pour l'administration à l'adventice du vaisseau,
dans lequel les microparticules sont dispersées dans ledit support liquide pour une utilisation en tant que médicament.

25. Composition pharmaceutique selon la revendication 24, dans laquelle les microparticules ont une taille allant jusqu'à 50 µm de diamètre.

26. Composition pharmaceutique selon la revendication 24 ou 25, dans laquelle l'agent thérapeutique lipophile est encapsulé à l'intérieur de microsphères.

27. Composition pharmaceutique selon la revendication 24 ou 25, dans laquelle l'agent thérapeutique lipophile est fixé à la surface extérieure de microsphères.

28. Composition pharmaceutique selon l'une quelconque revendications 24 ou 25, dans laquelle l'agent thérapeutique lipophile est formé en un dendrimère.

29. Composition pharmaceutique selon l'une quelconque des revendications 24 ou 25, dans laquelle l'agent thérapeutique lipophile est transporté sous la forme de molécules hôtes à l'intérieur d'un vide dendritique d'un dendrimère.

30. Composition pharmaceutique selon l'une quelconque des revendications 24 ou 25, dans laquelle les microparticules sont formées dans des globules qui comprennent la phase discontinue d'une émulsion.

31. Composition pharmaceutique selon l'une quelconque des revendications 24 à 30, dans laquelle le support liquide est une solution saline ou une autre solution aqueuse ou, pour une émulsion, un milieu de dispersion biocompatible.

32. Composition pharmaceutique selon l'une quelconque des revendications 24 à 31, dans laquelle l'agent thérapeutique est lipophile et le support liquide est une solution saline ou une solution aqueuse.
